Europäisches Patentamt

European Patent Office

Office européen des brevets

(11) Numéro de publication: **0 066 028**
**B1**

(12) **FASCICULE DE BREVET EUROPEEN**

(45) Date de publication du fascicule du brevet: **23.01.85**

(51) Int. Cl.⁴: **A 61 F 5/01**

(21) Numéro de dépôt: **81400949.4**

(22) Date de dépôt: **15.06.81**

(54) **Appareillage externe de station verticale et de marche pour handicapés moteurs des membres inférieurs.**

(30) Priorité: **01.06.81 FR 8110764**

(43) Date de publication de la demande:
**08.12.82 Bulletin 82/49**

(45) Mention de la délivrance du brevet:
**23.01.85 Bulletin 85/04**

(84) Etats contractants désignés:
**AT BE CH DE FR GB IT LI LU NL SE**

(56) Documents cités:
**US-A-1 336 695**
**US-A-1 660 721**
**US-A-2 654 365**
**US-A-2 772 674**

**MEDIZINISCH-ORTHOPAEDISCHE TECHNIK,
Vol. 101, No. 2, mars-avril 1981, Stuttgart R.O.
NITSCHKE "Laterale Hüft-Knie-Fuss-Orthesen",
pages 39 à 41**

**Le dossier contient des informations
techniques présentées postérieurement au
dépôt de la demande et ne figurant pas dans le
présent fascicule.**

(73) Titulaire: **Salort, Guy
219, rue Raymond Losserand
F-75014 Paris (FR)**

(72) Inventeur: **Salort, Guy
219, rue Raymond Losserand
F-75014 Paris (FR)**

(74) Mandataire: **Peuscet, Jacques
3, Square de Maubeuge
F-75009 Paris (FR)**

Courier Press, Leamington Spa, England.

## Description

La présente invention a trait à un appareillage externe de station verticale et de marche pour handicapés moteurs des membres inférieurs.

Plus précisément, le but de la présente invention est de fournir un tel appareillage qui permette la station verticale et la locomotion des handicapés moteurs des membres inférieurs au moyen d'un appareillage autonome constitué par un para-squelette amovible et léger permettant à la fois de pallier l'insuffisance du tonus postural et de permettre de reconstituer le mécanisme physiologique de l'appui podal et de la marche.

Dans le demande de brevet US—A—2 654 365, on a déjà décrit un appareillage permettant la station verticale et la locomotion des handicapés moteurs des membres inférieurs. Cet appareillage comporte un corset qui est relié à une armature porteuse rigide disposée de part et d'autre du membre handicapé. L'armature est reliée au membre par des colliers en cuir au niveau de la cuisse et de la jambe, et elle comporte une articulation au niveau du genou. L'armature porteuse est reliée par une articulation à rotule à une barre rigide faisant partie intégrante du corset. L'inconvénient essentiel d'un tel appareillage est de constituer une orthèse qui prend en charge tout le membre appareillé, de sorte que le membre ne peut pas participer de façon active au support du poids du corps, puisque l'ensemble du poids est supporté par l'armature porteuse rigide. En outre, un tel appareillage ne peut donner lieu qu'à un mouvement de marche pendulaire.

L'invention concerne un appareillage externe de station verticale et de marche pour handicapés moteurs d'un ou des deux membres inférieurs, ledit appareillage comportant, en combinaison:

— un corset monocoque disposé à hauteur de la ceinture pelvienne;
— une armature associée à chaque membre à appareiller, ladite armature comportant;
— d'une part, un élément fémoral externe disposé parallèlement à la face externe du fémur et sensiblement sur toute sa longueur, ledit élément ayant son extrémité supérieure reliée au corset, au voisinage de la crête iliaque, par un moyen, qui est disposé sensiblement dans le prolongement de l'élément et qui est fixé audit corset;
— et, d'autre part, un élément tibial, disposé du côté de lu face interne du tibia et sensiblement sur toute sa longueur, ledit élément étant relié à l'élément fémoral, au niveau du genou, par un organe de liaison permettant leurs mouvements relatifs; caractérisé par le fait que:
— l'élément fémoral externe est un levier-ressort comprenant une lame de métal flexible susceptible d'encaisser et restituer les contraintes de flexion et de torsion;
— l'élément tibial est un levier-ressort antéro-interne comprenant une lame de métal flexible susceptible d'encaisser et de restituer les contraintes de flexion et de torsion;
— l'organe de liaison est une genouillère susceptible de maintenir les mouvements relatifs entre la cuisse et la jambe en léger flexum et léger valgum, dans les limites étroites du jeu articulaire physiologique, grâce à des éléments élastiques disposés, du côté interne et du côté externe du genou, entre des zones de la genouillère situées au-dessus et au-dessous de l'articulation, tant sur la face frontale que sur la face postérieure;
— le moyen reliant le levier-ressort fémoral au corset est une première sangle élastique, ledit levier-ressort fémoral étant, en outre, relié au corset par une deuxième sangle élastique, qui s'étend transversalement en étant légèrement inclinée vers le haut pour se fixer sur le corset vers ou en direction des épines iliaques, ladite deuxième sangle croisant ainsi sensiblement la symphyse pubienne;
— la genouillère est reliée rigidement, d'une part, dans sa zone supérieure, à l'extrémité inférieure du levier-ressort fémoral et, d'autre part, dans sa zone inférieure, à l'extrémité supérieure du levier-ressort tibial;
— l'extrémité inférieure du levier-ressort tibial est reliée rigidement à un élément limitant le jeu articulaire de l'articulation tibio-tarsienne, telle qu'une chaussure.

Dans le cas où le handicap moteur porte sur un seul membre inférieur, l'appareillage ne comprend qu'un seul jeu d'éléments appliqués sur ce membre, en plus du corset. Si, au contraire, le handicap porte sur les deux membres inférieurs, chaque membre porte les éléments correspondants.

L'appareillage selon l'invention réalise, en fait, une véritable inversion du système physiologique du membre inférieur et se distingue donc par là radicalement des prothèses déjà développées, qui cherchent à assurer un soutien prenant en charge tout le membre, alors que dans l'invention, le membre participe de façon pour ainsi dire active au fonctionnement.

Grâce à l'appareillage selon l'invention, une marche automatique devient possible par le biais du déplacement volontaire du centre de gravité du corps en arrière et latéralement, à la façon d'un "moteur pelvien" pour reproduire le mécanisme naturel de la marche, qui n'est qu'une succession de chutes évitées. L'appareillage, par la modulation préalablement réglée de l'amplitude de ses mouvements, assure le recentrage de la charge corporelle selon l'axe du squelette du sujet et l'amortissement du mouvement moteur par inertie, afin de ne pas dépasser les limites du polygone de sustentation et d'éviter la chute.

Le corset monocoque est peu ou pas déformable afin d'assurer une semi-rigidité de la partie inférieure du tronc et permettre d'assurer le mouvement moteur par le déplacement de la partie du tronc située au-dessus du corset, ce dernier ayant également pour rôle d'améliorer la liaison entre la partie d'appareillage s'étendant parallèlement au fémur et le bassin, notamment au niveau des ailes iliaques.

De façon avantageuse, le levier ressort fémoral externe s'étend entre une extrémité supérieure sous-trochantérienne et une extrémité inférieure disposée sensiblement au-dessus du condyle fémoral.

Le levier-ressort tibial, en position antéro-interne, s'étend de façon avantageuse entre une extrémité supérieure disposés sous le plateau tibial et une extrémité inférieure sus-malléolaire.

Les moyens permettant à la genouillère de maintenir l'articulation du genou en léger flexum et léger valgum comportent, de préférence, un système de sangles élastiques convenablement disposées pour former des freins élastiques au récurvatum, au varum, au valgum et au flexum. Ces sangles sont, de préférence, réglables de façon à adapter la genouillère à chaque cas précis pour maintenir l'articulation normalement en léger flexum et léger valgum. Le flexum peut être, par exemple de l'ordre de 120 à 160° et, de façon avantageuse, être tel qu'à l'état de repos, lorsque le sujet est debout, le plateau tibial forme avec le plan horizontal un angle vers le bas et vers l'avant qui ne soit pas inférieur à 5°. De même, l'angle du plateau en valgum est, de préférence, égal ou supérieur à 5° par rapport à l'horizontale. La genouillère selon l'invention est également conçue pour constituer un frein aux rotations interne et externe et pour avoir tendance à favoriser la rotation interne stabilisant ainsi la tête fémorale pour assurer un recentrage à chaque pas, le membre inférieur étant, en fait, sollicité en vis interne lors de son mouvement.

D'autres avantages et caractéristiques de l'invention apparaîtront à la lecture de la description suivante, faite à titre d'exemple non limitatif et se référant au dessin annexé dans lequel:

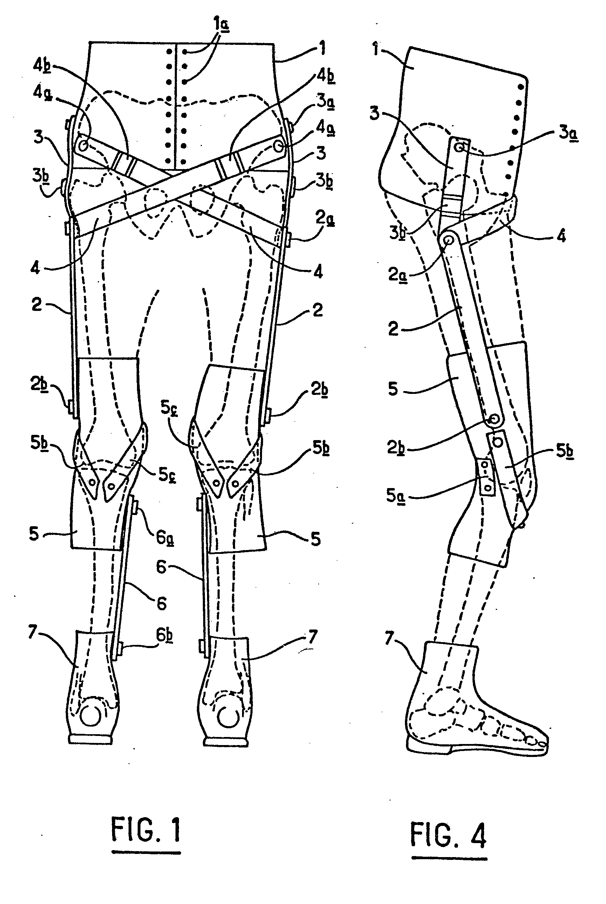

— la figure 1 représente une vue de face schématique de l'appareillage;

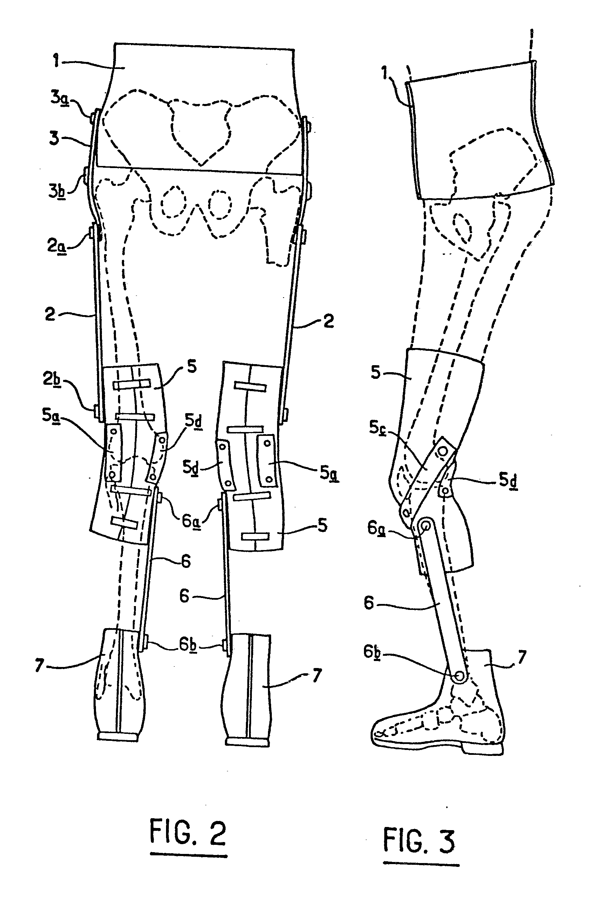

— la figure 2 représente une vue postérieure schématique de cet appareillage;
— la figure 3 représente une vue latérale interne de cet appareillage;
— la figure 4 représente une vue latérale externe de cet appareillage.

L'appareillage représenté comporte un corset monocoque 1 entourant le bassin et l'abdomen et suffisamment développé en hauteur pour assurer la liaison entre le centre de gravité corporel et les membres inférieurs. Ce corset peut avantageusement s'ouvrir par l'avant et peut être, à cet effet, muni de boutonnières 1a permettant la fermeture du corset à l'état lacé. Comme on le voit sur la figure, la ligne supérieure du corset est située à un niveau légèrement sus-ombilical alors que la ligne inférieure du corset passe sensiblement au niveau sus-tronchantérien. Chaque ressort fémoral 2 est constitué d'une lame rectiligne relativement rigide mais susceptible d'empêcher des contraintes de flexion et de torsion pour les restituer et faire en conséquence office d'un accumulateur d'énergie. La lame 2 est, par exemple, constituée en métal léger (duralumiun) et présente une épaisseur de 3 mm et une largeur de 25 mm. Elle s'étend sur la face externe de la cuisse, sensiblement parallèlement au fémur, son extrémité supérieure 2a étant légèrement sous-trochantérienne alors que son extrémité inférieure 2b aboutit sensiblement au-dessus du condyle fémoral.

L'extrémité supérieure 2a est reliée à l'extrémité inférieure d'une sangle élastique 3 dont l'extrémité supérieure est fixée en 3a au corset 1 au niveau de la crête iliaque, de préférence, sensiblement au niveau des tubérosités antérieures. L'extrémité 2a est encore reliée à une sangle élastique 4 légèrement ascendante et se dirigeant vers la hanche opposée pour être fixée au corset, par exemple, en 4a au niveau, par exemple, de l'épine iliaque.

Des moyens 3b, 4b sont prévus pour permettre l'adaptation et le réglage de la longueur des sangles.

Les différentes sangles élastiques 3, 4, le corset 1 et les leviers fémoraux 2 forment ainsi un moteur pelvien dont la fonctionnement sera décrit par la suite.

Les extrémités inférieures 2b des lames-ressorts 2 sont reliées à une genouillère 5 dont le rôle consiste, d'une part, à assurer une certaine transmission de forces entre les ressorts fémoraux et les ressorts tibiaux et, d'autre part, à contrôler les possibilités de déplacement articulaire de l'articulation du genou à l'intérieur de limites étroites du jeu physiologique.

A cette fin, cette genouillère 5 constituée, par exemple, en un matériau relativement souple, comporte des freins élastiques réalisés sous forme de sangles élastiques, de préférence, à longueur réglable, destinées à permettre un léger débattement articulaire et à assurer un confort à la marche. La première sangle 5a prolonge vers le bas l'extrémité 2b du ressort fémoral 2 en passant sur l'interligne articulaire fémoro-tibiale et en couvrant la face extéro-postérieure du condyle externe et la face latérale de plateau tibial. Cette sangle 5a constitue un frein au récurvatum et au varum.

La sangle 5b, plus longue, s'étend sur une ligne de force prolongeant l'extrémité inférieure 2b du ressort fémoral externe en passant en pont sur la face externe du condyle externe, l'interligne, le plateau tibial externe à l'extérieur du cône antérieur para-rotulien et s'insérant finalement en position sous-patellaire au regard du tendon rotulien. Cette sangle 5b constitue un frein au flexum et à la rotation interne et assure la stabilité externe du cône para-rotulien.

Le troisième frein est constitué par la sangle 5c qui s'étend depuis le tendon rotulien en remontant vers le haut et vers l'intérieur pour ponter l'interligne et la face interne du condyle intérieur pour se terminer sensiblement au-dessus et en arrière du condyle interne. C'est un frein au flexum, au valgum et à la rotation interne. Il assure la stabilité du cône para-rotulien.

Le quatrième frein 5d est tendu de la face interne du tibia au niveau de l'insertion du ressort tibial interne et s'étend vers le sommet postéreiur du condyle interne en couvrant sa face postérieure. Il est un frein à la rotation interne, au récurvatum et au valgum.

Ces différens freins sont réglés de façon à maintenir normalement le plateau tibial dans une position de flexum au moins égale à 5° par rapport à l'horizontale et en valgum de préférence de 5° par rapport à l'horizontale. En d'autres termes, cette genouillère positionne, imprime et régule un mouvement de vis interne sur le membre inférieur porteur. Le flexum permet de s'assurer que le poids du corps a toujours tendance à provoquer un glissement vers l'avant et le valgum pour obtenir par un mouvement en vis interne un recentrage de la charge à travers le membre inférieur.

Les réglages des différents freins 5a, 5d sont évidemment effectués en fonction de l'état du genou qui peut être lui-même déformé de façon permanente, notamment en varum et récurvatum.

Le ressort tibial 6 est fixé en 6a sur la genouillère 5 en position antéro-interne et s'étend le long de la diaphyse tibiale jusqu'en son extrémité 6b, au niveau de la maléole interne où il est fixé sur la tige montante d'une chaussure orthopédique 7.

On comprend donc que lorsque le sujet qui porte l'appareillage se trouve en position debout, comme représenté sur le dessin, le membre inférieur se trouve maintenu dans cette position par la chaussure orthopédique 7 et la genouillère 5 qui limitent les possibilités du jeu des articulations tibio-tarsienne et fémoro-tibiale, le maintien de la position entre la cuisse et le bassin restant assuré par le gaine, les sangles 3, 4 et la régulation céréballeuse de posture. On comprend cependant que dans cette position les ressorts fémoraux 2 et tibiaux 6, activés par le mouvement du tronc transmis par le corset, suppléent au manque de tonus musculaire, du fait que les mouvements relatifs à partir de la position représentée sur le dessin provoquent une déformation des ressorts qui exercent en réaction un effort inverse.

Dans cette position, l'ensemble mobile formé par le tronc, la tête et les membres supérieurs peut trouver deux points d'appui au niveau des têtes fémorales dans leurs cotyles. A partir de la position d'équilibre représentée sur le dessin, le déplacement du centre de gravité latéralement et en arrière n'est possible que si le point d'appui, qui doit être fémoral, recevant le déplacement progressif de la charge, est capable de fournir à cette charge une série de points d'appui dynamiques de résistance, de sens et de direction opposée. Lorsque par l'effet des déséquilibres volontaires ainsi créés, une des jambes quitte le sol, l'absorption du doublement de la charge sur l'autre membre inférieur s'effectue avec un maintien en équilibre grâce au levier flexible fémoral 2 et au levier flexible tibial interne 6 correspondant. Ceux-ci qui se trouvent ainsi déformés vont pouvoir ensuite restituer progressivement dans le temps et dans l'espace leurs contraintes lors du passage du poids sur l'autre appui, ce qui dégage une force de propulsion dirigée vers l'avant, en dedans et en bas. L'effet des sangles verticales latérales 3 est de limiter de hancher et de recentrer la charge et de permettre son ajustement sur la tête fémorale en effectuant un effort de traction sur les structures inférieures du para-squelette du membre correspondant et en augmentant également la compression en vis interne. Ainsi, à chaque déplacement latéral arrière du centre de gravité du tronc par rapport à la tête fémorale chargée de la jambe en appui, le cotyle pelvien correspondant va décrire, avec la charge qu'il supporte (à savoir le tronc et le membre inférieur non en appui), un mouvement circulaire vers l'arrière et l'extérieur, de sens et de direction opposé à celui de l'appui donné par la tête fémorale, qui se déplace vers l'avant et l'intérieur. Ce mouvement va être contrôlé par les sangles croisées 4; on a donc un ensemble composé de trois points ayant des positions relatives fixes, à savoir les deux épines iliaques antérieures et le pubis; cet ensemble est actionné par le levier volontaire supérieur, de sorte que les poussées vers l'avant du pubis, à chaque mouvement arrière du tronc, sont transmises automatiquement aux deux épines iliaques et donc aux deux têtes fémorales; il en résulte que ces poussées, dues au mouvement volontaire du tronc, sont transmises aux deux appuis dynamiques constituée par les ressorts fémoraux externes grâce à l'allongement élastique des sangles croisées 4. Ceci crée un ensemble travaillant comme un différentiel autour d'un axe qui passe par l'une ou l'autre des articulations coxo-fémorales (à savoir celle du membre d'appui au sol), ladite articulation étant actionnée et chargée par le levier volontaire supérieur, ce qui crée un moteur pelvien à inertie permettant la marche.

**Revendications**

1. Appareillage externe de station verticale et de marche pour handicapé moteur d'un (ou des deux) membre(s) inférieur(s), ledit appareillage comportant, en combinaison:

— un corset monocoque (1) disposé à hauteur de la ceinture pelvienne;
— une armature associée à chaque membre à appareiller, ladite armature comportant;
— d'une part, un élément fémoral externe (2) disposé parallèlement à la face externe du fémur et sensiblement sur toute sa longueur, ledit élément ayant son extrémité supérieure reliée au corset (1), au voisinage de la crête iliaque, par un moyen (3), qui est disposé sensiblement dans le prolongement de l'élément (2) et qui est fixé audit corset (1);
— et, d'autre part, un élément tibial (6), disposé du côté de la face interne du tibia et sensiblement sur toute sa longueur, ledit élément étant relié à l'élément fémoral (2), au niveau du genou, par un organe de liaison (5) permettant leurs mouvements relatifs; caractérisé par le fait que:
— l'élément fémoral externe est un levier-ressort (2) comprenant une lame de métal flexible susceptible d'encaisser et restituer les contraintes de flexion et de torsion;
— l'élément tibial est un levier-ressort antéro-interne (6) comprenant une lame de métal flexible susceptible d'encaisser et de restituer les contraintes de flexion et de torsion;
— l'organe de liaison est une genouillère (5) susceptible de maintenir les mouvements relatifs entre la cuisse et la jambe en léger flexum et léger valgum, dans les limites étroites du jeu articulaire physiologique, grâce à des éléments élastiques (5a, 5b, 5c, 5d) disposés, du côté interne et du côté externe du genou, entre des zones de la genouillère situées au-dessus et au-dessous de l'articulation, tant sur la face frontale que sur la face postérieure;
— le moyen reliant le levier-ressort fémoral (2) au corset (1) est une première sangle élastique (3), ledit levier-ressort fémoral (2) étant, en outre, relié au corset (1) par une deuxième sangle élastique (4), qui s'étend transversalement en étant légèrement inclinée vers le haut pour se fixer (en 4a) sur le corset (1) vers on en direction des épines iliaques, ladite deuxième sangle (4) croisant ainsi sensiblement la symphyse pubienne;
— la genouillère (5) est reliée rigidement, d'une part, dans sa zone supérieure, à l'extrémité inférieure (2b) du levier-ressort fémoral (2) et, d'autre part, dans sa zone inférieure, à l'extrémité supérieure (6a) du levier-ressort tibial (6);
— l'extrémité inférieure du levier-ressort tibial (6) est reliée rigidement à un élément (7) limitant le jeu articulaire de l'articulation tibio-tarsienne, telle qu'une chaussure.

2. Appareillage selon la revendication 1, caractérisé par le fait que les sangles (3, 4) sont de longueuer réglable.
3. Appareillage selon l'une des revendications 1 et 2, caractérisé par le fait que le levier-ressort fémoral (2) s'étend depuis une extrémité supérieure (2a) sensiblement sous-trochantérienne vers une extrémité inférieure (2b) sensiblement sus-condylienne.
4. Appareillage selon l'une des revendications 1 à 3, caractérisé par le fait que le levier-ressort tibial (6) s'étend entre une extrémité supérieure (6a) sensiblement sous-articulaire et une extrémité inférieure (6b) sensiblement sus-malléolaire.
5. Appareillage selon l'une des revendications 1 à 4, caractérisé par le fait que la genouillère (5) comporte une pluralité de sangles (5a, 5b, 5c, 5d) constituant des freins de récurvatum, varum, valgum, flexum, ainsi qu'aux rotations interne et externe.
6. Appareillage selon la revendication 5, caractérisé par le fait que les sangles (5a, 5b, 5c, 5d) sont de longueur réglable.

**Patentansprüche**

1. Die aufrechte Haltung und das Gehen ermöglichende Exoprothese für einen Bewegungsbehinderten an einem unteren Glied oder an beiden unteren Gliedmaßen, wobei die Prothese in Kombination aufweist:

— ein selbsttragendes Korsett (1), das auf der Höhe des Beckengürtels angeordnet ist;
— ein Gestell, das jedem zu prothetisierendem Glied zugeordnet ist, wobei das Gestell aufweist:
— einerseits ein äußeres Oberschenkelelement (2), das parallel zur Außenfläche des Oberschenkels und im wesentlichen auf seiner ganzen Länge angeordnet ist, wobei das Element mit seinem oberen Ende mit dem Korsett (1) in der Nähe des Darmbeinkamms durch eine Einrichtung (3) verbunden ist, die im wesentlichen in der Verlängerung des Elements (2) angeordnet und an dem Korsett (1) befestigt ist,
— und andererseits ein Schienbeinelement (6), das auf der Seite der Innenfläche des Schienbeins und im wesentlichen auf seiner ganzen Länge angeordnet ist, wobei dieses Element mit dem Oberschenkel (2) auf der Höhe des Knies durch ein Verbindungsorgan (5) verbunden ist, welches Relativbewegungen ermöglicht, dadurch gekennzeichnet, daß
— das externe Oberschenkelelement ein Federhebel (2) ist, der eine dünne Leiste aus flexiblem Metall aufweist, die in der Lage ist, Biegebeanspruchungen und Torsionsbeanspruchungen aufzunehmen und abzugeben;
— das Schienbeinelement ein vorderer innere Federhebel (6) ist, der eine dünne Leiste aus flexiblem Metall aufweist, die in der Lage ist, Biegebeanspruchungen und Torsionsbeanspruchungen aufzunehmen und abzugeben;
— das Verbindungsorgan eine Kniebandage (5) ist, die in der Lage ist, Relativbewegungen zwischen dem Schenkel und dem Bein in leichtem Flexum und leichtem Valgum in den engen Grenzen des physiologischen Gelenkspiels aufgrund von elastischen Elementen (5a, 5b, 5c, 5d) beizubehalten, die auf der Innenseite und der Außenseite des Knies zwischen den Zonen der Kniebandage angeordnet sind, die über und unter dem Gelenk sowohl auf der Vorderseite als auch auf der Rückseite liegen;

— die Einrichtung, welche den Oberschenkelfederhebel (2) mit dem Korsett (1) verbindet, ein erster elastischer Gurt (3) mit, wobei der Oberschenkelfederhebel (2) außerdem mit dem Korsett (1) durch einen zweiten elastischen Gurt (4) verbunden ist, der sich transversal unter einer leichten Niegung nach oben für seine Befestigung (in 4a) auf dem Korsett (1) zu den Darmbeindornen oder in deren Richtung erstreckt, wobei der zweite Gurt (4) so im wesentlichen die Schambeinfuge kreuzt;

— die Kniebandage (5) starr einerseits in ihrer oberen Zone mit dem unteren Ende (2b) des Oberschenkelfederhebels (2) und andererseits in ihrer unteren Zone mit dem oberen Ende (6a) des Schienbeinfederhebels (6) verbunden ist;

— das untere Ende des Schienbeinfederhebels (6) starr mit einem Element (7) verbunden ist, welches das Gelenkspiel des Schienbein-Fußwurzelgelenks begrenzt, wie einem Schuh.

2. Prothese nach Anspruch 1, dadurch gekennzeichnet, daß die Länge der Gurte (3, 4) einstellbar ist.

3. Prothese nach einem der Ansprüche 1 und 2, dadurch gekennzeichnet, daß der Oberschenkelfederhebel (2) sich von einem oberen Ende (2a) im wesentlichen unterhalb des Rollhügels zu einem unteren Ende (2b) im wesentlichen über dem Gelenkkopf erstreckt.

4. Prothese nach einem der Ansprüche 1 bis 3, dadurch gekennzeichnet, daß der Schienbeinfederhebel (6) sich zwischen einem oberen Ende (6a) im wesentlichen unter dem Gelenk und einem unteren Ende (6b) im wesentlichen über dem Knöchel erstreckt.

5. Prothese nach einem der Ansprüche 1 bis 4, dadurch gekennzeichnet, daß die Kniebandage (5) eine Vielzahl von Gurten (5a, 5b, 5c, 5d) aufweist, die Rekurvatum-, Varum- Valgum-, Flexum-Widerstände bzw. -Bändchen bilden sowie für interne und externe Drehbewegungen.

6. Prothese nach Anspruch 5, dadurch gekennzeichnet, daß die Länge der Gurte (5a, 5b, 5c, 5d) einstellbar ist.

**Claims**

1. An external apparatus for the vertical stance and for walking for the disabled with motor handicaps of one (or of two) lower limb (or limbs), the said apparatus comprising in combination;

— a single piece corset (1) disposed at the level of the pelvic girdle;

— a brace associated with each limb to be equipped, the said brace comprising;

— on the one hand, an external femoral element (2) disposed parallel to the external side of the femur and substantially over all its length, the said element having its upper end joined to the corset (1) near the iliac crest by a means (3) which is disposed substantially in the extension of element (2) and which is fixed to the said corset (1);

— and, on the other hand, a tibial element (6) disposed on the inner side of the tibia and substantially over its whole length, the said element being joined to the femoral element (2) at the level of the knee, by a joint element (5) allowing their relative movements; characterised in that:

— the external femoral element is a spring lever (2) comprising a strip of flexible metal capable of absorbing and returning the flexural and torsional stresses;

— the tibial element is an antero-internal spring lever (6) comprising a strip of flexible metal capable of absorbing and returning the flexural and torsional stresses;

— the joint element is a knee cap (5) capable of maintaining the relative movements between the thigh and the leg in slight flexion and slight valgus within the narrow limits of the physiological articular play by means of the elements (5a, 5b, 5c, 5d) disposed on the internal side and the external side of the knee between the zones of the knee cap situated above and below the joint, both on the frontal side and on the posterior side;

— the means joining the femoral spring lever (2) to corset (1) is a first elastic strap (3), the said femoral spring lever (2) being moreover, connected to corset (1) via a second elastic strap (4) which extends transversely whilst being slightly inclined towards the top to be fixed (at 4a) on corset (1) towards or in the direction of the iliac spines, the said second strap (4) substantially crossing the pubic symphisis;

— the knee cap (5) is rigidly connected on the one hand in its upper zone to the lower end (2b) of the femoral spring lever (2) and, on the other hand, in its lower zone, to the upper end (6a) of the tibial spring lever (6);

— the lower end of the tibial spring lever (6) is rigidly connected to an element (7) restricting the articular play of the tibial-tarsal articulation, such as a boot.

2. An apparatus according to Claim 1, characterised in that the straps (3, 4) are of adjustable length.

3. An apparatus according to one of Claims 1 and 2, characterised in that the femoral spring lever (2) extends from a substantially subtrochanteric upper end (2a) towards a lower end (2b) substantially above the condyle.

4. An apparatus according to one of Claims 1 to 3, characterised in that the tibial spring lever (6) extends between an upper end (6a) substantially below the joint and a lower end (6b) substantially above the malleolus.

5. An apparatus according to one of Claims 1 to 4, characterised in that the knee cap (5) comprises a plurality of straps (5a, 5b, 5c, 5d) constituting restraints to recurvation, varus, valgus, flexion, as well as to internal and external rotations.

6. An apparatus according to Claim 5, characterised in that the straps (5a, 5b, 5c, 5d) are of adjustable length.

FIG. 1

FIG. 4

FIG. 2          FIG. 3